# FASCICULE DE BREVET EUROPEEN

(11) **EP 0 412 869 B1**
(45) Date de publication et mention de la délivrance du brevet: **27.04.1994**
(21) Numéro de dépôt: 90402157.3
(22) Date de dépôt: 26.07.1990
(51) Int. Cl.: A61L 15/26, A61L 15/44, A61L 15/60

(54) **Film composite pour traitement de surface et procédés de fabrication correspondants**
Filmzusammensetzung zur Behandlung von Oberflächen und Verfahren zu ihrer Herstellung
Composite film for surface treatment and its corresponding methods of manufacture

(30) Priorité: 11.08.1989 FR 8910825
(43) Date de publication de la demande: 13.02.1991
(73) Titulaire: L'OREAL, 75008 Paris (FR)
(72) Inventeur: Gueret, Jean-Louis, F-75018 Paris (FR); Lebreton, Françoise, F-91440 Bures-Sur-Yvette (FR); Contamin, Jean-Claude, F-91420 Morangis (FR)
(74) Mandataire: Peuscet, Jacques

(56) Documents cités:
- EP-A- 0 137 278
- EP-A- 0 184 910
- EP-A- 0 196 769
- EP-A- 0 224 981
- EP-A- 0 289 342
- EP-A- 0 309 309
- GB-A- 2 100 605

## Description

La présente invention concerne un film composite pour traitement local d'une surface, notamment d'une zone de peau, et des procédés de fabrication dudit film.

Il est connu d'appliquer sur la peau des films permettant, par transdermie, de faire pénétrer dans la peau des produits pharmaceutiques et/ou cosmétiques traitants, notamment de l'eau ou des solutions aqueuses.

On a déjà proposé, notamment dans la demande de brevet européen EP-A- 0 285 563, d'utiliser à cet effet, des films composites comportant plusieurs couches successives dans l'ordre suivant : une première couche, dite occlusive, est généralement constituée par une feuille de substance imperméable aux produits traitants de façon à empêcher leur évaporation et faciliter la transdermie ; une seconde couche, dite couche réservoir, contient le(s) produit(s) traitant(s) : cette couche est généralement mise en contact avec la peau et, pour faciliter la fixation du film composite sur la peau, on enduit la couche réservoir d'une couche de substance adhésive perméable aux produits de traitement. Enfin, ces films composites sont souvent protégés, au cours du stockage, par une pellicule détachable, que l'on peut enlever par pelage au moment de l'utilisation et qui est disposée du côté opposé à celui où se trouve la couche occlusive.

Dans ces films composites, on a tenté d'utiliser, comme couche réservoir, une couche de silicone contenant, sous forme de dispersion, des micro-gouttelettes d'une phase aqueuse, en particulier d'eau. Lorsque le film composite est appliqué sur la peau, la couche occlusive permet de condenser de l'eau dans la couche réservoir ; on facilite ainsi le transfert à travers la peau de l'eau et des autres substances actives contenues dans la phase aqueuse. Malheureusement l'eau s'évapore rapidement hors d'une telle couche réservoir et a pratiquement disparu au bout de quelques heures. Il est donc impossible de conserver et stocker ces films. On a déjà proposé de réduire en grande partie l'évaporation de l'eau, en introduisant dans la couche de silicone à la fois la phase aqueuse et au moins un agent gélifiant de cette phase aqueuse (voir à cet égard, EP-A-0 137 278). Cependant, pour des tels films, lorsque la couche réservoir est chargée d'une phase aqueuse gélifiée dispersée dans la matrice polymère, on a jusqu'alors toujours été obligé d'utiliser une couche adhésive pour assurer la fixation du film sur la peau pour le traitement par transdermie. En effet, dès que la quantité de phase aqueuse gélifiée dépasse environ 10 % en poids dans la couche réservoir, il devient pratiquement impossible de produire une matrice polymère de silicone qui soit auto-adhésive grâce au caractère partiel de la polymérisation de la matrice silicone et qui, néanmoins, ait une résistance mécanique suffisante pour éviter sa fragmentation au cours de son emploi. Or l'utilisation d'une couche adhésive sur la couche réservoir freine la transdermie même si l'adhésif est susceptible de laisser passer l'eau et requiert une étape supplémentaire dans le processus de fabrication, ce qui augmente le coût de revient du produit.

La demanderesse propose un film composite, qui permet d'éviter les inconvénients précités. Dans ce film, la matrice de polymère silicone contient jusqu'à 50 % en poids de phase aqueuse gélifiée par rapport au poids total de la couche réservoir et elle n'est que partiellement polymérisée pour lui assurer son caractère auto-adhésif ; sa résistance mécanique est assurée grâce à une trame qui y est incluse et qui constitue une armature de la couche réservoir : l'ouverture de maille de la trame est suffisante pour ne pas gêner le passage de la phase aqueuse destinée au traitement à réaliser mais assez restreinte pour former néanmoins une barrière entre la surface traitée et les inclusions de gel et permettre ainsi l'auto-adhésivité de la matrice sur la surface à traiter. Les dimensions respectives de la trame, de la couche réservoir et des inclusions de gel sont définies pour que l'on ait simultanément un bon effet traitant, une manipulation aisée et une auto-adhésivité suffisante du film composite selon l'invention.

La présente invention a donc pour objet un film composite pour traitement local d'une surface comportant, d'une part, une couche occlusive et, d'autre part, une couche réservoir, qui renferme une phase aqueuse active et est associée à une protection amovible, la couche réservoir étant constituée par une matrice formée d'un polymère de silicone à l'intérieur de laquelle sont disposées des inclusions constituées par la phase aqueuse active gélifiée grâce à au moins un agent gélifiant, caractérisé par le fait que :
a) la matrice de polymère de silicone n'est que partiellement réticulée pour être auto-adhésive sur la surface à traiter ;
b) la phase aqueuse active gélifiée représente de 10 à 50 % en poids du poids total de la couche réservoir ;
c) la couche réservoir a une épaisseur comprise entre 0,2 et 4 mm ;
d) la dimension moyenne des inclusions de phase aqueuse active gélifiée est choisie entre 0,05 et 3 mm, cette dimension moyenne étant au plus égale à l'épaisseur de la couche réservoir ;
e) la face de la couche réservoir, qui est opposée à la couche occlusive, porte une trame renforçant mécaniquement ladite couche réservoir, ladite trame étant incluse dans la couche réservoir et définissant des mailles de dimension moyenne choisie entre 0,01 mm et la dimension moyenne des inclusions, ladite trame comportant de 10 à 1.000 mailles par centimètre carré et ayant une épaisseur comprise entre 0,01 et 1,5 mm, ladite épaisseur étant au plus égale à l'épaisseur de la couche réservoir.

La présence de gélifiant dans la phase aqueuse ralentit très nettement l'évaporation de l'eau hors de la couche réservoir et le film composite préparé avec cette couche réservoir peut être stocké pendant un temps suffisant dans les conditions courantes d'utilisation. De plus, en présence d'agent gélifiant, on peut introduire une quantité d'eau supérieure dans la couche réservoir. Par exemple, en présence d'alcool polyvinylique, on peut introduire dans la matrice silicone de la couche réservoir 60 % d'eau en poids alors qu'en l'absence d'agent gélifiant on ne peut pas dépasser 30 % d'eau en poids. On pense, bien que cette explication ne soit aucunement limitative, que cette augmentation de la quantité de phase aqueuse stockée provient du fait que, si la cellule d'une inclusion est rompue au cours de la manipulation du film ou ouverte en raison de son positionnement en bordure de la couche réservoir, la phase aqueuse ne s'écoule pas hors de la cellule où elle se trouve à cause de la gélification des inclusions, ce qui réduit les pertes et évite le déssèchement de la couche réservoir à partir de ses bordures.

Par polymère de silicone, on entend dans la présente demande, des organopolysiloxanes linéaires substitués sur l'atome de Si par des radicaux choisis dans le groupe formé par des radicaux alkyle en C₁ - C₆ , aryle ou ar(alkyle C₁ - C₂), les atomes de silicium terminaux étant tri-substitués. Des organopolysiloxanes de ce type ont été décrits notamment dans les brevets US-A-2 541 137, 2 723 966, 2 863 846, 2 890 188, 2 927 907, 3 002 951 et 3 035 016.

Dans un mode préféré de mise en oeuvre du film composite selon l'invention, la surface traitée par ledit film est une zone de peau et la phase aqueuse active contient au moins un actif cosmétique et/ou pharmaceutique.

La phase aqueuse peut être constituée par de l'eau ou par un mélange eau/propylène glycol. Elle peut également être une solution dans l'eau d'au moins une substance cosmétiquement et/ou pharmaceutiquement active hydrosoluble. La phase aqueuse peut également être constituée par une émulsion ou une dispersion d'un produit non soluble dans l'eau, par exemple une huile, qui peut contenir des substances actives liposolubles. Parmi les substances cosmétiquement et/ou pharmaceutiquement actives hydrosolubles ou liposolubles que l'on peut introduire dans la phase aqueuse, on peut citer, de façon non limitative :
- les facteurs d'oxygénation cellulaire tels que le D-panthénol, l'extrait de rate, la guanosine, les thymus peptides, les oligo-éléments ;
- les actifs régénérants, nourrissants et/ou cicatrisants tels que l'hydroxyproline, les extraits de levure, les extraits placentaires, les facteurs de croissance, la centella asiatica, les extraits d'embryons ou autres extraits biologiques, et les cérébrosides, sphingocéryls ou autres lipides de type céramides ;
- les actifs raffermissants tels que le lactate de monométhyl-silanol, les mannuronates, le triméthyl silanol et l'alchenille ;
- les produits filmogènes et/ou tenseurs tels que le liquide amniotique, le sérum albumine, et le sérum de cheval ;
- les produits hydratants tels que la glycérine, l'urée, le lactate de sodium, les mucopolysaccharides, les acides aminés, l'acide hyaluronique, le filagrinol, la chitine et les dérivés de chitosane, les peptides, l'ADN, le collagène, l'élastine ;
- les vitamines hydrosolubles telles que les vitamines C, B2, H, panthénol, B6, PP, l'acide folique, l'ascorbyl-2 phosphate de magnésium ;
- les vitamines liposolubles ou leurs dérivés tels que les vitamines A, E, F, le palmitate de vitamine A, le rétinol ;
- les anti-inflammatoires tels que l'acide glycyrrhétinique, l'alpha-bisabolol, l'allantoïne, l'acide palmityl collagénique, ou l'acide acétylsalicylique ;
- les extraits végétaux, par exemple de lierre, d'algue, de bouleau, de ruscus ou petit houx, de ginko biloba, de ginseng, de prêle ou d'aloès ;
- les actifs blanchissants tels que l'hydroquinone ou l'acide kojique ;
- et d'autres actifs spécifiques divers, tels que les bases xanthiques comme la caféine, le nicotinate d'alpha tocophérol,
   le nicotinate de méthyle, le menthol, le camphre, l'acide asiatique, l'escine, les acides alicycliques ou les thioxolones.

L'agent gélifiant est, de préférence, un agent gélifiant qui gonfle à l'eau. Parmi les agents gélifiants utilisables selon l'invention, on peut citer les substances naturelles telles que les amidons, les gommes naturelles (gomme de guar, gomme arabique, gomme tragacanthe), la caséine, les phytocolloïdes (carragénates, alginates, agar-agar) et, de préférence, la gélatine :
- les dérivés semi-synthétiques de la cellulose tels que la carboxyméthylcellulose ;
- les polymères synthétiques tels que l'alcool polyvinylique ; les polymères vinyliques solubles dans l'eau, par exemple les produits vendus sous la marque CARBOPOL par la Société GOODRICH et, de préférence, les polyacrylates réticulés superabsorbants à fort taux de gonflement dans l'eau, par exemple les produits commercialisés sous la marque AQUA KEEP par la société NORSOLOR.

La matrice de silicone contient des inclusions de phase aqueuse sous forme dispersée. La dimension des inclusions est réglée par action sur l'agitation mise en oeuvre pour le mélange des ingrédients, au cours du processus de fabrication de la couche réservoir.

La couche réservoir contient également, de façon connue, un catalyseur de réticulation du silicone.

La couche réservoir peut en outre contenir des particules hydrophiles dispersées d'origine végétale ou minérale ; ces particules peuvent être des fibres ou des grains de poudre. La couche réservoir peut contenir jusqu'à 50 % en poids de particules hydrophiles par rapport au poids du polymère de silicone. L'intérêt de l'introduction des particules hydrophiles dans la matrice de la couche réservoir est que l'on favorise ainsi, par capillarité, la sortie de la phase aqueuse qui assure le traitement de la surface sur laquelle le film selon l'invention est appliquée.

Selon l'invention, la couche réservoir contient une trame imputrescible, qui renforce mécaniquement ladite couche. Cette trame est constituée par une feuille de matériau plastique perforé, par un non-tissé perforé, ou par un filet tissé, le non-tissé ou le filet étant constitué de fibres naturelles ou synthétiques. Elle est, par exemple, constituée par un filet en polyamide comme décrit dans FR-A-2 620 914.

La couche réservoir peut être préparée en mélangeant sous agitation la phase aqueuse active déjà gélifiée dans un polymère de silicone non réticulé. Elle peut être également préparée en mélangeant sous agitation un silicone non réticulé, la phase aqueuse active et l'agent gélifiant. Le mélange sous agitation est effectué soit entre 1 et 5°C pour éviter un début de réticulation du polymère de silicone, soit à température ambiante, soit également à une température à laquelle l'agent gélifiant est à l'état liquide. Le mélange destiné à former la couche réservoir contient un catalyseur de réticulation et on chauffe ledit mélange à une température comprise entre 25 et 150°C pendant un temps compris entre 1 min. et 120 min. On choisit la quantité de catalyseur de réticulation et le chauffage de façon à obtenir une réticulation incomplète du silicone de la couche réservoir afin qu'elle bénéficie d'une auto-adhésivité satisfaisante.

Le film composite selon l'invention comporte en plus de la couche réservoir définie ci-dessus une couche occlusive. Ladite couche occlusive est, de préférence, une feuille de matériau thermoplastique avec ou sans élastomère, en particulier une feuille de chlorure de polyvinyle, de copolymère d'éthylène et d'acétate de vinyle, de polyéthylène, de polyester ou de polyuréthanne. Cette feuille peut être gaufrée ou microperforée. Elle est, de préférence, traitée "anti-glissant" pour améliorer l'adhérence entre la couche occlusive et la couche réservoir. Il est particulièrement intéressant de choisir de réaliser la couche occlusive au moyen d'une feuille de polyéthylène, ce qui est possible étant donné que la matrice de polymère de silicone est suffisamment peu réticulée pour être auto-adhésive : l'intérêt de l'utilisation du polyéthylène provient du fait que ce matériau présente une bonne imperméabilité à l'eau, de sorte qu'au cours du stockage, le film composite selon l'invention, ne présente aucun dessèchement de la couche réservoir, sous réserve de protéger celle-ci de façon convenable, du côté opposé à la couche occlusive.

Selon un premier mode de réalisation, la protection amovible du film composite selon l'invention, est une pellicule de protection détachable qui recouvre la surface réservoir sur sa face opposée à la couche occlusive. Selon un autre mode de réalisation, la protection amovible associée au film composite selon l'invention, est un récipient contenant la phase aqueuse active, le sous-ensemble constitué par la couche occlusive et la couche réservoir étant plongé dans ledit récipient pour obtenir le film utilisable pour le traitement, la couche réservoir dudit sous-ensemble avant son introduction dans le récipient ne comportant que des inclusions constituées d'agents gélifiants non chargés de phase aqueuse active.

Dans le cas où la couche réservoir est protégée par une pellicule de protection détachable ou pelable, cette pellicule protège ladite couche réservoir pendant le stockage et les différentes manipulations du produit ; on l'enlève au moment de l'utilisation. Cette pellicule de protection peut être constituée par une feuille de papier siliconée ou par une feuille de matériau thermoplastique traitée de façon à la rendre anti-adhérente, par exemple à l'aide d'un vernis. Selon une réalisation particulièrement préférée, la pellicule de protection est réalisée en polyéthylène, de même que la couche occlusive. Dans un tel cas, on fait en sorte que la pellicule de protection ait une épaisseur supérieure à celle de la couche occlusive de façon à être plus rigide pour assurer un décollement plus facile au moment de la mise en oeuvre du produit. Ce décollement est d'autant plus aisé que la pellicule de protection est en appui sur la couche réservoir du côté où se trouve la trame de la couche réservoir ce qui réduit la surface d'adhérence de la pellicule de protection sur la matrice de polymère de silicone auto-adhésive. On a constaté que l'on obtenait des résultats particulièrement satisfaisants lorsque la couche occlusive avait une épaisseur comprise entre 0,01 et 0,1 mm et lorsque la pellicule de protection avait une épaisseur comprise entre 0,05 et 0,3 mm, l'épaisseur de la pellicule de protection étant comprise entre 1,5 fois et 3 fois l'épaisseur de la couche occlusive.

De façon connue, le film composite selon l'invention, peut être découpé selon un contour approprié correspondant à la zone de surface à traiter; les bordures dudit contour sont alors de préférence comprimées pour chasser la majeure partie de la couche réservoir et mettre sensiblement en contact la couche occlusive et la pellicule de protection. Dans le cas où la couche occlusive a une épaisseur inférieure à la pellicule de protection, comme il a été ci-dessus indiqué, la compression entraîne une déformation préférentielle de la couche occlusive, étant donné que la pellicule de protection est plus rigide ; il en résulte que le produit a une face d'application plane c'est à dire que les bords de la couche occlusive se trouvent sensiblement dans le même plan que la face de la couche réservoir qui vient en contact avec la peau : il en résulte une sensation de confort à l'application et une amélioration de l'isolement vis-à-vis de l'extérieur de la zone traitée, ce qui est favorable pour l'exécution d'une transdermie.

Pour l'application sur le corps humain, le film composite selon l'invention peut, de façon connue, être découpé sous forme de masque pour l'application sur le visage, sous forme de demi-couronne pour l'application sur le buste, sous forme de pastille ou sous toute autre forme nécessaire pour l'application en une zone déterminée du corps.

Selon un mode de mise en oeuvre avantageux, le polymère de silicone contenant ses inclusions, un catalyseur de réticulation et, éventuellement, d'autres additifs, est maintenu sous agitation permanente dans une trémie, à une température de 50° à 60°C. Le mélange est délivré à l'aide d'une pompe ou d'un robinet, devant une racle sous laquelle passe la pellicule de protection détachable et la trame, provenant par exemple chacune d'un rouleau. Par dessus, avant calandrage, on fait venir la feuille de couche occlusive provenant également d'un rouleau on règle, en passant sous une calandre, l'épaisseur de la couche réservoir qui a été préformée par la racle en amont. Le film composite obtenu passe ensuite dans un tunnel à air chaud, dans lequel la température et la vitesse de passage du film composite sont ajustées de façon à obtenir un temps de polymérisation convenable.

Pour obtenir un produit constitué d'un film composite selon l'invention, on peut utiliser par exemple, un premier procédé selon lequel on dispose sur la pellicule de protection la trame de la couche réservoir, on couche sur cet ensemble le mélange formé par le polymère de silicone comportant ses inclusions de phase aqueuse gélifiée, on pose sur la couche ainsi constituée la feuille constitutive de la couche occlusive, on découpe le film composite de façon que ses bords aient un contour correspondant à la zone de surface à traiter, on comprime les bords du film découpé pour chasser, dans la zone de compression, la majeure partie du mélange constitutif de la couche réservoir et on chauffe le produit pour provoquer la réticulation partielle du polymère de silicone de la couche réservoir.

Mais selon un autre procédé, on introduit et on disperse dans le polymère de silicone de la couche réservoir, un agent gélifiant constitué d'un polyacrylate réticulé super-absorbant à l'état non-hydraté, on couche ce mélange sur la feuille constitutive de la couche occlusive, on met en place la trame du film composite, on provoque la réticulation partielle, du polymère de silicone de la couche réservoir et on plonge le film ainsi obtenu dans une phase aqueuse active contenue dans un récipient, ledit récipient constituant la protection du film composite.

### EXEMPLE

A 2 g de polyacrylate en poudre (produit commercialisé sous la dénomination commerciale "AQUAKEEP 10 SHNP" commercialisé par la société "NORSOLOR"), on ajoute 40 g d'une solution aqueuse de D-panthénol à 10% en poids. On mélange lentement au moyen d'un agitateur à pales perforées. Ensuite, on ajoute 100 g d'organopolysiloxane (vendu par la société "DOW CORNING" sous la dénomination commerciale "Silastic MDX 4-4210 medical grade"). Sous agitation à 1000 tours/min., on ajoute 6 g de catalyseur "Silastic MDX 4-4210 medical grade curing agent" vendu par la société "DOW CORNING", et on maintient l'agitation pendant 10 min.

Le produit ainsi homogénéisé est étalé en couche de 0,5 mm d'épaisseur sur une feuille de polyéthylène d'une épaisseur de 0,2 mm. Cette pellicule de protection a été traitée préalablement en surface par une huile siliconée pour réduire son adhérence. Sur la pellicule de protection est mise en place avant le couchage une trame constituée par un filet en polyamide comportant environ 20 mailles/cm², ayant une dimension moyenne de maille de 0,4 mm et une épaisseur de 0,1 mm.

Par dessus le couchage, on pose un film de polyéthylène (sans traitement glissant) de 0,04 mm d'épaisseur qui constitue la couche occlusive et on chauffe cet ensemble dans une étuve ventilée pendant 3 min. à 90°C.

Après refroidissement, on obtient ainsi un film composite comportant une couche occlusive et une couche réservoir formée d'une matrice en polymère de silicone partiellement polymérisé, l'ensemble étant muni d'une pellicule de protection qui, lorsqu'on la retire, permet l'adhésion de la matrice de polymère de silicone sur la peau. La pellicule de protection est enlevée sans aucune difficulté ; après usage, le film est séparé sans aucune difficulté de la peau sur laquelle il était appliqué ; ces deux résultats sont dus au renfort que constitue la trame de la couche réservoir et ce, malgré le faible degré de polymérisation de la matrice de polymère de silicone.

Le film composite est découpé en morceaux de taille souhaitée et appliqué, par une légère pression, sur les zones de la peau affectées, par exemple, de dermatoses ou de brûlures. De préférence, on applique ce film composite le soir, les actifs étant libérés pendant la nuit, et on retire la matrice épuisée le matin.

## Revendications

1. Film composite pour traitement local d'une surface comportant, d'une part, une couche occlusive, et, d'autre part, une couche réservoir, qui renferme une phase aqueuse active et est associée à une protection amovible, la couche réservoir étant constituée par une matrice formée d'un polymère de silicone à l'intérieur de laquelle sont disposées des inclusions constituées par la phase aqueuse active gélifiée grâce à au moins un agent gélifiant, caractérisé par le fait que :
a) la matrice de polymère de silicone n'est que partiellement réticulée pour être auto-adhésive sur la surface à traiter ;
b) la phase aqueuse active gélifiée représente de 10 à 50 % en poids du poids total de la couche réservoir ;
c) la couche réservoir a une épaisseur comprise entre 0,2 et 4 mm ;
d) la dimension moyenne des inclusions de phase aqueuse active gélifiée est choisie entre 0,05 et 3 mm, cette dimension moyenne étant au plus égale à l'épaisseur de la couche réservoir ;
e) la face de la couche réservoir, qui est opposée à la couche occlusive, porte une trame renforçant mécaniquement la-dite couche réservoir, ladite trame étant incluse dans la couche réservoir et définissant des mailles de dimension moyenne choisie entre 0,01 mm et la dimension moyenne des inclusions, ladite trame comportant de 10 à 1.000 mailles par centimètre carré et ayant une épaisseur comprise entre 0,01 et 1,5 mm, ladite épaisseur étant au plus égale à l'épaisseur de la couche réservoir.

2. Film selon la revendication 1, caractérisé par le fait que la surface traitée par ledit film est une zone de peau, et que la phase aqueuse active contient au moins un actif cosmétique et/ou pharmaceutique.

3. Film selon la revendication 2, caractérisé par le fait que la phase aqueuse gélifiée est constituée par un produit pris dans le groupe formé par l'eau, un mélange eau/propylène glycol, une solution aqueuse d'au moins une substance hydrosoluble cosmétiquement et/ou pharmaceutiquement active, une émulsion ou une dispersion dans l'eau d'une substance cosmétiquement et/ou pharmaceutiquement active non hydrosoluble.

4. Film selon la revendication 3 dans lequel la phase aqueuse gélifiée est une émulsion ou une dispersion dans l'eau d'une substance active non hydrosoluble, caractérisé par le fait que ladite substance active non hydrosoluble est une huile pouvant contenir des actifs liposolubles.

5. Film selon l'une des revendications 1 à 4, caractérisé par le fait que l'agent gélifiant est un agent qui gonfle en présence d'eau.

6. Film selon la revendication 5, caractérisé par le fait que l'agent gélifiant est une substance naturelle, un dérivé synthétique de la cellulose ou un polymère synthétique.

7. Film selon la revendication 6, caractérisé par le fait que l'agent gélifiant est la gélatine.

8. Film selon la revendication 6, caractérisé par le fait que l'agent gélifiant est un polyacrylate réticulé super-absorbant.

9. Film selon l'une des revendications 1 à 8, caractérisé par le fait que la matrice formée d'un polymère de silicone contient des particules hydrophiles dispersées d'origine végétale ou minérale.

10. Film selon la revendication 9, caractérisé par le fait que les particules hydrophiles sont des fibres ou des grains de poudre.

11. Film selon l'une des revendications 9 ou 10, caractérisé par le fait que la matrice formée d'un polymère de silicone contient jusqu 'à 50 % en poids de particules hydrophiles par rapport au poids du polymère de silicone.

12. Film selon l'une des revendications 1 à 11, caractérisé par le fait que la protection amovible, qui lui est associée est une pellicule de protection détachable qui recouvre la couche réservoir sur sa face opposée à la couche occlusive.

13. Film selon l'une des revendications 1 à 11, caractérisé par le fait que la protection amovible, qui lui est associée est un récipient contenant la phase aqueuse active, le sous-ensemble constitué par la couche occlusive et la couche réservoir étant plongé dans ledit récipient pour obtenir le film utilisable pour le traitement, la couche réservoir dudit sous-ensemble avant son introduction dans le récipient ne comportant que des inclusions constituées d'agent gélifiant non chargé de phase aqueuse active.

14. Film selon la revendication 12, caractérisé par le fait que la couche occlusive et la pellicule de protection sont formées d'une feuille de polymère thermoplastique, avec ou sans élastomère.

15. Film selon la revendication 14, caractérisé par le fait que le polymère thermoplastique est choisi dans le groupe formé par le chlorure de polyvinyle, les copolymères d'éthylène et d'acétate de vinyle, les polyéthylènes, les polyesters et les polyuréthannes.

16. Film selon la revendication 15, caractérisé par le fait que la couche occlusive et la pellicule de protection sont constituées l'une et l'autre par une feuille de polyéthylène.

17. Film selon la revendication 16, caractérisé par le fait que la couche occlusive a une épaisseur comprise entre 0,01 et 0,1 mm et que la pellicule de protection a une épaisseur comprise entre 0,05 et 0,3 mm, l'épaisseur de la pellicule de protection étant comprise entre 1,5 fois et 3 fois l'épaisseur de la couche occlusive.

18. Film selon la revendication 16, caractérisé par le fait qu'il est découpé selon un contour approprié correspondant à la zone de surface à traiter, les bordures dudit contour ayant été comprimées pour chasser la majeure partie de la couche réservoir et mettre sensiblement en contact la couche occlusive et la pellicule de protection.

19. Film selon l'une des revendications 1 à 18, caractérisé par le fait que la trame de la couche réservoir est constituée par une feuille de matériau plastique perforé, par un non-tissé perforé, ou par un filet tissé, le non-tissé ou le filet étant constitué de fibres naturelles ou synthétiques.

20. Procédé de fabrication d'un film selon l'une des revendications 1 à 19, caractérisé par le fait que l'on prépare la couche réservoir en mélangeant sous agitation la phase aqueuse active déjà gélifiée dans un polymère silicone non réticulé.

21. Procédé de fabrication d'un film selon l'une des revendications 1 à 19, caractérisé par le fait que l'on prépare la couche réservoir en mélangeant sous agitation un polymère de silicone non réticulé, une phase aqueuse active et un agent gélifiant.

22. Procédé selon l'une des revendications 20 ou 21, caractérisé par le fait que l'on effectue le mélange sous agitation à température ambiante.

23. Procédé selon l'une des revendications 20 ou 21, caractérisé par le fait que l'on effectue le mélange sous agitation à une température à laquelle l'agent gélifiant est à l'état liquide.

24. Procédé selon l'une des revendications 20 à 23, caractérisé par le fait que, dans le mélange destiné à former la couche réservoir, on introduit un catalyseur de réticulation et que l'on chauffe ledit mélange à une température comprise entre 25 et 150°C pendant un temps compris entre 1 minute et 120 minutes.

25. Procédé selon la revendication 24, caractérisé par le fait que l'on réalise sous agitation le mélange destiné à former la couche réservoir à une température comprise entre 1 et 5°C pour éviter le début de réticulation du polymère de silicone.

26. Procédé selon l'une des revendications 24 ou 25, caractérisé par le fait que l'on choisit la quantité de catalyseur de réticulation et le chauffage de façon à obtenir une réticulation incomplète du silicone de la couche réservoir.

27. Procédé selon l'une des revendications 20 à 26, caractérisé par le fait que l'on dispose sur la pellicule de protection la trame de la couche réservoir, que l'on couche sur cet ensemble le mélange formé par le polymère de silicone comportant ses inclusions de phase aqueuse gélifiée, que l'on pose sur la couche ainsi constituée la feuille constitutive de la couche occlusive, que l'on découpe le film composite de façon que ses bords aient un contour correspondant à la zone de surface à traiter, que l'on comprime les bords du film découpé pour chasser, dans la zone de compression, la majeure partie du mélange constitutif de la couche réservoir et que l'on chauffe le produit pour provoquer la réticulation partielle du polymère de silicone de la couche réservoir.

28. Procédé de fabrication d'un film composite selon la revendication 8, caractérisé par le fait que dans le polymère de silicone de la couche réservoir, on introduit et on disperse l'agent gélifiant à l'état non-hydraté, que l'on couche ce mélange sur la feuille constitutive de la couche occlusive, que l'on met en place la trame du film composite, que l'on provoque la réticulation partielle du polymère de silicone de la couche réservoir, et que l'on plonge le film ainsi obtenu dans une phase aqueuse active contenu dans un récipient, ledit récipient constituant la protection du film composite.

## Patentansprüche

1. Kompositfilm zur lokalen Behandlung einer Oberfläche, umfassend eine Okklusivschicht und eine Reservoirschicht, welche eine aktive, wäßrige Phase einschließt und mit einem entfernbaren Schutz assoziiert ist, wobei die Reservoirschicht aus einer von einem Silikonpolymer gebildeten Matrix besteht, in deren Inneren Einschlüsse ausgebildet sind, welche aus der mit Hilfe wenigstens eines gelbildenden Mittels gebildeten aktiven, wäßrigen Gelphase bestehen,
**dadurch gekennzeichnet,** daß:
a) die Silikonpolymer-Matrix nur partiell vernetzt ist, so daß sie auf der zu behandelnden Oberfläche selbstklebend ist;
b) der Anteil der aktiven, wäßrigen Gelphase 10 bis 50 Gew.-%, bezogen auf das Gesamtgewicht der Reservoirschicht, beträgt;
c) die Reservoirschicht eine Dicke im Bereich von 0,2 bis 4 mm aufweist;
d) die mittlere Abmessung der Einschlüsse in der aktiven, wäßrigen Gelphase einen Wert von 0,05 bis 3 mm einnimmt, wobei diese mittlere Abmessung höchstens gleich der Dicke der Reservoirschicht ist; und
e) diejenige Seite der Reservoirschicht, die der Okklusivschicht gegenüberliegt, eine Einlage enthält, welche die Reservoirschicht mechanisch verstärkt, wobei die Einlage in der Reservoirschicht eingeschlossen ist und Maschen definiert, deren mittlere Größe ausgewählt ist zwischen 0,01 mm und der mittleren Größe der Einschlüsse, wobei die Einlage 10 bis 1 000 Maschen pro Quadratzentimeter umfaßt und eine Dicke im Bereich von 0,01 und 1,5 mm aufweist, wobei die Dicke höchstens gleich der Dicke der Reservoirschicht ist.

2. Film nach Anspruch 1, dadurch gekennzeichnet, daß die mit diesem Film behandelte Oberfläche ein Hautabschnitt ist, und daß die wäßrige aktive Phase wenigstens einen kosmetisch und/oder pharmazeutisch aktiven Bestandteil enthält.

3. Film nach Anspruch 2, dadurch gekennzeichnet, daß die wäßrige Gelphase von einem Produkt gebildet wird, das ausgewählt ist unter Wasser, einem Wasser/Propylenglykol-Gemisch, einer wäßrigen Lösung wenigstens einer wasserlöslichen kosmetisch und/oder pharmazeutisch aktiven Substanz, einer Emulsion oder einer Dispersion einer wasserunlöslichen, kosmetisch und/oder pharmazeutisch aktiven Substanz in Wasser.

4. Film nach Anspruch 3, wobei die wäßrige Gelphase eine Emulsion oder eine Dispersion einer aktiven, wasserunlöslichen Substanz in Wasser ist, dadurch gekennzeichnet, daß die aktive wasserunlösliche Substanz ein Öl ist, das gegebenenfalls fettlösliche aktive Bestandteile enthält.

5. Film nach einem der Ansprüche 1 bis 4, dadurch gekennzeichnet, daß das gelbildende Mittel ein Mittel ist, das in Gegenwart von Wasser quillt.

6. Film nach Anspruch 5, dadurch gekennzeichnet, daß das gelbildende Mittel eine natürliche Substanz, ein synthetisches Cellulosederivat oder ein synthetisches Polymer ist.

7. Film nach Anspruch 6, dadurch gekennzeichnet, daß das das gelbildende Mittel Gelatine ist.

8. Film nach Anspruch 6, dadurch gekennzeichnet, daß das gelbildende Mittel ein hochabsorbierendes vernetztes Polyacrylat ist.

9. Film nach einem der Ansprüche 1 bis 8, dadurch gekennzeichnet, daß die von einem Silikonpolymer gebildete Matrix dispergierte hydrophile Partikel pflanzlichen oder mineralischen Ursprungs enthält.

10. Film nach Anspruch 9, dadurch gekennzeichnet, daß die hydrophilen Partikel Fasern oder Pulverkörner sind.

11. Film nach einem der Ansprüche 9 oder 10, dadurch gekennzeichnet, daß die von einem Silikonpolymer gebildete Matrix bis zu 50 Gew.-% hydrophile Partikel, bezogen auf das Gewicht des Silikonpolymers, enthält.

12. Film nach einem der Ansprüche 1 bis 11, dadurch gekennzeichnet, daß der damit assoziierte, entfernbare Schutz ein abnehmbarer Schutzfilm ist, der die Reservoirschicht auf ihrer, der Okklusivschicht gegenüberliegenden Seite bedeckt.

13. Film nach einem der Ansprüche 1 bis 11, dadurch gekennzeichnet, daß der damit assoziierte entfernbare Schutz ein Behälter ist, der die wäßrige aktive Phase enthält, wobei die von der Okklusivschicht und der Reservoirschicht gebildete Untereinheit in den Behälter verlängert ist, um einen zur Behandlung verwendbaren Film zu erhalten, wobei die Reservoirschicht dieser Untereinheit vor ihrem Einbringen in den Behälter nur Einschlüsse umfaßt, die aus gelbildendem Mittel bestehen und nicht mit aktiver wäßriger Phase beladen sind.

14. Film nach Anspruch 12, dadurch gekennzeichnet, daß die Okklusivschicht und der Schutzfilm von einem thermoplastischen, gegebenfalls Elastomer-haltigen Polymerfilm gebildet werden.

15. Film nach Anspruch 14, dadurch gekennzeichnet, daß das thermoplastische Polymer ausgewählt ist unter Polyvinylchlorid, Ethylen-Vinylacetatcopolymeren, Polyethylenen, Polyestern und Polyurethanen.

16. Film nach Anspruch 15, dadurch gekennzeichnet, daß die Okklusivschicht und der Schutzfilm jeweils von einer Polyethylenfolie gebildet werden.

17. Film nach Anspruch 16, dadurch gekennzeichnet, daß die Okklusivschicht eine Dicke im Bereich von 0,01 bis 0,1 mm aufweist und daß der Schutzfilm eine Dicke im Bereich von 0,05 bis 0,3 mm aufweist, wobei die Dicke des Schutzfilms dem 1,5-fachen bis 3-fachen der Dicke der Okklusivschicht entspricht.

18. Film nach Anspruch 16, dadurch gekennzeichnet, daß dessen Zuschnitt einen dem Abschnitt der zu behandelnden Oberfläche entsprechenden Umriß aufweist, wobei die Ränder des Umrisses komprimiert sind, um den Hauptteil der Reservoirschicht einzufassen und um die Okklusivschicht und den Schutzfilm im wesentlichen in Kontakt zu bringen.

19. Film nach einem der Ansprüche 1 bis 18, dadurch gekennzeichnet, daß die Einlage in der Reservoirschicht aus einer Folie aus performiertem Kunststoffmaterial, aus einem perforierten Faservlies, oder aus einem Netzgewebe besteht, wobei das Faserflies oder das Netz aus natürlichen oder synthetischen Fasern hergestellt sind.

20. Verfahren zur Herstellung eines Films nach einem der Ansprüche 1 bis 19, dadurch gekennzeichnet, daß man die Reservoirschicht herstellt, indem man unter Rühren die bereits gelierte, wäßrige, aktive Phase mit einem unvernetzten Silikonpolymer vermischt.

21. Verfahren zur Herstellung eines Films nach einem der Ansprüche 1 bis 19, dadurch gekennzeichnet, daß man die Reservoirschicht herstellt, indem man unter Rühren ein unvernetztes Silikonpolymer, eine wäßrige aktive Phase und ein gelbildendes Mittel vermischt.

22. Verfahren nach einem der Ansprüche 20 oder 21, dadurch gekennzeichnet, daß man zum Mischen bei Umgebungstemperatur rührt.

23. Verfahren nach einem der Ansprüche 20 oder 21, dadurch gekennzeichnet, daß man zum Mischen bei einer Temperatur rührt, bei der sich das gelbildende Mittel im flüssigen Zustand befindet.

24. Verfahren nach einem der Ansprüche 20 bis 23, dadurch gekennzeichnet, daß man in die zur Ausbildung der Reservoirschicht bestimmte Mischung einen Vernetzungskatalysator einführt und daß man diese Mischung auf eine Temperatur im Bereich von 25 bis 150°C über einen Zeitraum von 1 Minute bis 120 Minuten erwärmt.

25. Verfahren nach Anspruch 24, dadurch gekennzeichnet, daß man die zur Ausbildung der Reservoirschicht bestimmte Mischung unter Rühren bei einer Temperatur im Bereich von 1 bis 5°C herstellt, um den Start der Vernetzungsreaktion des Silikonpolymers zu verhindern.

26. Verfahren nach einem der Ansprüche 24 oder 25, dadurch gekennzeichnet, daß man die Menge des Vernetzungskatalysators und die Erwärmung so wählt, daß man eine unvollständige Vernetzung des Silikons der Reservoirschicht erhält.

27. Verfahren nach einem der Ansprüche 20 bis 26, dadurch gekennzeichnet, daß man auf dem Schutzfilm die Einlage für die Reservoirschicht anordnet, daß man darauf die von dem Silikonpolymer gebildete Mischung, die als Einschlüsse die wäßrige Gelphase enthält, aufträgt, daß man auf die so gebildete Schicht die die Okklusiivschicht bildende Folie legt, daß man den Kompositfilm in der Weise zuschneidet, daß seine Ränder einen dem Abschnitt der zu behandelnden Oberfläche entsprechenden Umriß aufweisen, daß man die Ränder des zugeschnittenen Films komprimiert, um in dem komprimierten Abschnitt den Hauptteil der die Reservoirschicht bildenden Mischung einzufassen, und daß man das Produkt erwärmt, um die partielle Vernetzung des Silikonpolymers der Reservoirschicht zu bewirken.

28. Verfahren zur Herstellung eines Kompositfilms nach Anspruch 8, dadurch gekennzeichnet, daß man in das Silikonpolymer der Reservoirschicht ein nichthydratisiertes gelbildendes Mittel einführt und dispergiert, daß man diese Mischung auf die die Okklusivschicht bildende Folie aufträgt, daß man die Einlage des Kompositfilms anordnet, daß man die partielle Vernetzung des Silikonpolymers der Reservoirschicht bewirkt, und daß man den auf diese Weise erhaltenen Film in einen die wäßrige, aktive Phase enthaltenden Behälter eintaucht, wobei der Behälter den Kompositfilm-Schutz bildet.

## Claims

1. Composite film for local treatment of a surface comprising, on the one hand, an occlusive layer and, on the other hand, a reservoir layer which encloses an active aqueous phase and is coupled to a removable protection, the reservoir layer consisting of a matrix made up of a silicone polymer inside which are arranged inclusions consisting of the active aqueous phase gelled by virtue of at least one gelling agent, characterized in that:
a) the silicone polymer matrix is only partially crosslinked, to be self-adhesive to the surface to be treated;
b) the gelled active aqueous phase represents from 10 to 50 % by weight of the total weight of the reservoir layer;
c) the reservoir layer has a thickness of between 0.2 and 4 mm;
d) the mean size of the gelled active aqueous phase inclusions is chosen between 0.05 and 3 mm, this mean size being at most equal to the thickness of the reservoir layer;
e) the face of the reservoir layer which is opposite the occlusive layer carries a web reinforcing the said reservoir layer mechanically, the said web being included in the reservoir layer and defining meshes of mean size chosen between 0.01 mm and the mean size of the inclusions, the said web comprising from 10 to 1000 meshes per square centimetre and having a thickness of between 0.01 and 1.5 mm, the said thickness being at most equal to the thickness of the reservoir layer.

2. Film according to claim 1, characterized in that the surface treated using the said film is a region of skin, and that the active aqueous phase contains at least one cosmetic and/or pharmaceutical agent.

3. Film according to claim 2, characterized in that the gelled aqueous phase consists of a product taken from the group made up of water, a water/propylene glycol mixture, an aqueous solution of at least one cosmetically and/or pharmaceutically active water-soluble substance, and an emulsion or a dispersion in water of a water-insoluble cosmetically and/or pharmaceutically active substance.

4. Film according to claim 3, in which the gelled aqueous phase is an emulsion or a dispersion in water of a water-insoluble active substance, characterized in that the said water-insoluble active substance is an oil which may contain liposoluble agents.

5. Film according to one of claims 1 to 4, characterized in that the gelling agent is an agent which swells in the presence of water.

6. Film according to claim 5, characterized in that the gelling agent is a natural substance, a synthetic cellulose derivative or a synthetic polymer.

7. Film according to claim 6, characterized in that the gelling agent is gelatine.

8. Film according to claim 6, characterized in that the gelling agent is a superabsorbent crosslinked polyacrylate.

9. Film according to one of claims 1 to 8, characterized in that the matrix made up of a silicone polymer contains dispersed hydrophilic particles of plant or mineral origin.

10. Film according to claim 9, characterized in that the hydrophilic particles are fibres or grains of powder.

11. Film according to either of claims 9 and 10, characterized in that the matrix made up of a silicone polymer contains up to 50 % by weight of hydrophilic particles relative to the weight of the silicone polymer.

12. Film according to one of claims 1 to 11, characterized in that the removable protection which is coupled with it is a removable protective surface film which covers the reservoir layer on its face opposite the occlusive layer.

13. Film according to one of claims 1 to 11, characterized in that the removable protection coupled with it is a receptacle containing the active aqueous phase, the subassembly consisting of the occlusive layer and the reservoir layer being immersed in the receptacle to obtain the film which can be employed for the treatment, the reservoir layer of the said subassembly before its introduction into the receptacle comprising only inclusions consisting of gelling agent carrying no active aqueous phase.

14. Film according to claim 12, characterized in that the occlusive layer and the protective surface film are made up of a sheet of thermoplastic polymer, with or without elastomer.

15. Film according to claim 14, characterized in that the thermoplastic polymer is chosen from the group made up of polyvinyl chloride, copolymers of ethylene and vinyl acetate, polyethylenes, polyesters and polyurethanes.

16. Film according to claim 15, characterized in that the occlusive layer and the protective surface film both consist of a polyethylene sheet.

17. Film according to claim 16, characterized in that the occlusive layer has a thickness of between 0.01 and 0.1 mm and that the protective surface film has a thickness of between 0.05 and 0.3 mm, the thickness of the protective surface film being between 1.5 times and 3 times the thickness of the occlusive layer.

18. Film according to claim 16, characterized in that it is cut out according to a suitable contour corresponding to the surface region to be treated, the edges of the said contour having been compressed to expel most of the reservoir layer and to bring the occlusive layer and the protective surface film substantially into contact.

19. Film according to one of claims 1 to 18, characterized in that the web of the reservoir layer consists of a sheet of perforated plastic material, of a perforated nonwoven, or of a woven net, the nonwoven or the net consisting of natural or synthetic fibres.

20. Process for the manufacture of a film according to one of claims 1 to 19, characterized in that the reservoir layer is prepared by mixing, with stirring, the already gelled active aqueous phase into an uncrosslinked silicone polymer.

21. Process for the manufacture of a film according to one of claims 1 to 19, characterized in that the reservoir layer is prepared by mixing, with stirring, an uncrosslinked silicone polymer, an active aqueous phase and a gelling agent.

22. Process according to either of claims 20 and 21, characterized in that the mixing is carried out with stirring at room temperature.

23. Process according to either of claims 20 and 21, characterized in that the mixing is carried out with stirring at a temperature at which the gelling agent is in the liquid state.

24. Process according to one of claims 20 to 23, characterized in that a crosslinking catalyst is introduced into the mixture intended to form the reservoir layer and the said mixture is heated to a temperature of between 25 and 150°C for a period of between 1 minute and 120 minutes.

25. Process according to claim 24, characterized in that the mixture intended to form the reservoir layer is produced with stirring at a temperature of between 1 and 5°C to avoid the onset of crosslinking of the silicone polymer.

26. Process according to either of claims 24 and 25, characterized in that the quantity of crosslinking catalyst and the heating are chosen so as to obtain an incomplete crosslinking of the silicone of the reservoir layer.

27. Process according to one of claims 20 to 26, characterized in that the web of the reservoir layer is arranged on the protective surface film, that the mixture made up of the silicone polymer comprising its gelled aqueous phase inclusions is coated onto this assembly, that the sheet constituting the occlusive layer is placed on the layer thus formed, that the composite film is cut out so that its edges have a contour corresponding to the surface region to be treated, that the edges of the cut-out film are compressed to expel, in the compression region, most of the mixture constituting the reservoir layer and that the product is heated to cause the partial crosslinking of the silicone polymer of the reservoir layer.

28. Process for the manufacture of a composite film according to claim 8, characterized in that the gelling agent in the unhydrated state is introduced into and dispersed in the silicone polymer of the reservoir layer, that this mixture is coated onto the sheet constituting the occlusive layer, that the web of the composite film is placed in position, that a partial crosslinking of the silicone polymer of the reservoir layer is produced, and that the film thus obtained is immersed in an active aqueous phase contained in a receptacle, the said receptacle constituting the protection of the composite film.
